# EUROPÄISCHE PATENTSCHRIFT

(11) **EP 1 914 549 B1**
(45) Veröffentlichungstag und Bekanntmachung des Hinweises auf die Patenterteilung: **20.01.2010**
(21) Anmeldenummer: 06022002.7
(22) Anmeldetag: 20.10.2006
(51) Int. Cl.: G01N 33/53, G01N 33/543, G01N 33/558

(54) **Verfahren und Vorrichtung zum Bestimmen der Konzentrationen von Liganden**
Method and device for determining ligand concentrations
Procédé et dispositif destinés à la détermination de la concentration de ligands

(43) Veröffentlichungstag der Anmeldung: 23.04.2008
(73) Patentinhaber: Micronas Holding GmbH, 79108 Freiburg (DE)
(72) Erfinder: Klapproth, Holger, Dr., 79108 Freiburg (DE)
(74) Vertreter: Huwer, Andreas

(56) Entgegenhaltungen:
- EP-A- 0 603 958
- EP-A1- 0 702 233
- WO-A-92/18866
- WO-A-98/41871
- WO-A-2005/052548
- WO-A-2006/084068
- US-A1- 2004 214 254
- US-B1- 6 197 503
- HOLLIGER PHILIPP ET AL: "Engineered antibody fragments and the rise of single domains." NATURE BIOTECHNOLOGY SEP 2005, Bd. 23, Nr. 9, September 2005 (2005-09), Seiten 1126-1136, XP008076746 ISSN: 1087-0156

## Beschreibung

Die Erfindung betrifft ein Verfahren nach dem Oberbegriff von Anspruch 1 und eine Vorrichtung nach dem Oberbegriff von Anspruch 12.

Eine derartige Vorrichtung ist aus US 6 107 503 B1 bekannt. Sie weist eine Durchfluss-Messkammer auf, die an ihrer Unterseite durch ein etwa plattenförmiges Substrat begrenzt ist, auf dem mehrere Teststellen angeordnet sind, an denen Rezeptoren immobilisiert sind. Die Rezeptoren sind jeweils für einen bestimmten, in einer zu analysierenden Probe enthaltenden Liganden bindungsspezifisch. An der Rückseite des Substrats ist ein Halbleiterchip vorgesehen, der an den einzelnen Teststellen jeweils einen optischen Sensor aufweist Zur Anregung der Emission von Lumineszenzstrahlung in Abhängigkeit von der Bindung der Liganden an die Rezeptoren weist die Vorrichtung eine optische Strahlungsquelle auf, in deren Abstrahlbereich die Rezeptoren angeordnet sind. Zum Messen der Konzentration der Liganden wird die Probe durch eine Einlassöffnung hindurch derart in die Messkammer eingebracht, dass die in der Probe enthaltenen Liganden an die Rezeptoren binden können. Nach Ablauf einer vorbestimmten Einwirkungsdauer werden nicht an einen Rezeptor gebundene Bestandteile der Probe aus der Messkammer entfernt. Die Einwirkungsdauer wird so gewählt, dass nach dem Entfernen der ungebundnen Bestandteile nur ein Teil der an den einzelnen Teststellen jeweils vorhandenen Bindungsstellen eines Rezeptors an den für den betreffenden Rezeptor bindungsspezifischen Liganden gebunden ist. Dabei ist das Verhältnis der gebundenen Bindungsstellen zu den freien Bindungsstellen von der Konzentration des betreffenden Liganden in der Probe abhängig.

In einem weiteren Verfahrensschritt wird eine Lösung in die Messkammer eingebracht, die Nachweisantikörper enthält, die mit einem Marker markiert sind. Die Nachweisantikörper sind für die Liganden bindungsspezifisch und binden an diese. Danach wird die Lösung aus der Messkammer entfernt, um die Teststellen mit Hilfe der Strahlungsquelle mit der Anregungsstrahlung zu bestrahlen. Die über die Liganden indirekt an die Rezeptoren gebundenen Nachweisantikörper werden durch die Anregungsstrahlung zur Aussendung einer Lumineszenzstrahlung angeregt, deren Wellenlänge sich von der Wellenlänge der Anregungsstrahlung unterscheidet. Mit Hilfe der optischen Sensoren, die für die Lumineszenzstrahlung empfindlich und für die Anregungsstrahlung unempfindlich sind, wird für jede Teststelle jeweils ein von der Lumineszenzstrahlung und somit der Konzentration der Liganden in der Probe abhängiges Messsignal erzeugt.

Die Vorrichtung hat den Nachteil, dass sie nur in einem begrenzten Konzentrationsbereich eine gleichzeitige Bestimmung der einzelnen Konzentrationswerte der unterschiedlichen, in der Probe enthaltenen Liganden ermöglicht. Bei bestimmten Proben, wie zum Beispiel Blutproben, kann es jedoch vorkommen, dass die in der Probe enthaltenen Liganden Konzentrationsunterschiede von bis zu sechs Dekaden aufweisen. Damit bei einer solchen Probe sowohl für den Liganden mit der geringsten Konzentration als auch für den Liganden mit der höchsten Konzentration jeweils ein Konzentrationswert bestimmt werden kann, ohne dass es an einer der Teststellen alle vorhandenen Rezeptor-Bindungsstellen an einen Liganden binden und somit der entsprechende Messwert in die Begrenzung gerät, werden mindestens zwei Versuche durchgeführt. Bei einem ersten, zur Bestimmung der Konzentration des den höchsten Konzentrationswert aufweisenden Liganden vorgesehenen Versuch wird eine geringe Einwirkungsdauer und bei einem zweiten, zur Bestimmung der Konzentration des den niedrigsten Konzentrationswert aufweisenden Liganden vorgesehenen Versuch, wird eine wesentlich größere Einwirkungsdauer gewählt als bei dem ersten Versuch. Nachteilig ist dabei, dass für jeden Versuch jeweils ein neuer Halbleiterchip benötigt wird, weshalb die Messung noch relativ teuer und arbeitsaufwändig ist. Ungünstig ist außerdem, dass eine entsprechend große Probenmenge benötigt wird. Bei der Untersuchung von Blutproben, die mit Hilfe eines Stechwerkzeugs aus dem Finger eines Patienten entnommen wird, kann dies bedeuten, dass an mindestens zwei Stellen Blut aus dem Finger entnommen werden muss, was besonders bei Kindern problematisch ist.

Aus WO 2006/084068 A2 und WO 2005/052548 A2 ist jeweils ein Verfahren zum Bestimmen der Konzentrationen von Liganden in einer zu analysierenden Probe bekannt, bei dem die Probe Liganden einer ersten Ligandenart aufweist, der eine erste Bindungsstelle haben, für die Rezeptoren einer ersten Rezeptorart bindungsspezifisch sind. Die Probe enthält außerdem Liganden einer zweiten Ligandenart, die eine zweite Bindungsstelle haben, für die Rezeptoren einer zweiten Rezeptorart bindungsspezifisch sind. Die Liganden der ersten Ligandenart sind in einer höheren Konzentration in der Probe enthalten als die Liganden der zweiten Ligandenart. Zum Wegfangen von Liganden der zweiten Ligandenart wird ein Antikörper-Fragment, das für die zweite Bindungsstelle bindungsspezifisch ist, derart mit der Probe vermischt wird, dass es in einer vorgegebenen Konzentration in dem so erhaltenen Gemisch vorliegt.

Ein erster Rezeptor der ersten Rezeptorart wird an einer ersten Teststelle und ein zweiter Rezeptor der zweiten Rezeptorart an einer zweiten Teststelle auf einem Substrat immobilisiert. Danach wird das Gemisch zum Binden der Liganden an die Rezeptoren derart mit dem Substrat in Kontakt gebracht, dass mindestens ein Ligand der ersten Ligandenart an den mindestens einen ersten Rezeptor und wenigstens ein Ligand der zweiten Ligandenart an den zumindest einen zweiten Rezeptor binden. Dann werden ein von der Konzentration des an den ersten Rezeptor gebundenen Liganden abhängiges erstes Messsignal und ein von der Konzentration des an den zweiten Rezeptor gebundenen Liganden abhängiges zweites Messsignal erzeugt. Mit Hilfe des ersten Messsignals wird die Konzentration des Liganden der ersten Ligandenart ermittelt. Die Konzentration der Liganden der zweiten Ligandenart wird mit Hilfe des zweiten Messsignals und der Konzentration des mindestens einen Antikörper-Fragments in dem Gemisch bestimmt.

Durch dieses Verfahren soll es möglich sein, für eine Vielzahl von Ligandenarten gleichzeitig Konzentrationsmesswerte zu messen, wobei hohe und geringe Konzentrationen gleichermaßen gemessen werden können. Das Verfahren hat jedoch den Nachteil, dass insbesondere bei Liganden, die jeweils mehrere Bindungsstellen haben, die an einen zweiten Rezeptoren binden können, beim Vermischen der Antikörper-Fragmente mit der Probe die Liganden mit den in Lösung befindlichen zweiten Rezeptoren vernetzen bzw. polymerisieren können. Der Dynamikbereich der Konzentrationsmessung reduziert sich dadurch erheblich.

Es besteht deshalb die Aufgabe, eine Vorrichtung und ein Verfahren der eingangs genannten Art zu schaffen, die eine einfache, schnelle und kostengünstige, aber dennoch genaue Messung der Konzentrationen mehrerer Liganden ermöglichen, die in unterschiedlicher Konzentration in einer Probe enthalten sind.

Diese Aufgabe wird bezüglich des Verfahrens mit den Merkmalen des Anspruchs 1 gelöst.

Das mindestens eine Antikörper-Fragment bindet den Liganden der zweiten Ligandenart so, dass dieser ganz oder teilweise an der Bindung an den zweiten Rezeptor gehindert wird. Auf diese Weise kann dann der Anteil der Liganden, die nicht - oder bei polyvalenten Liganden nicht vollständig - blockiert sind, an den zweiten Rezeptor binden. In vorteilhafter Weise ist es dadurch möglich, bei Proben, die mehrere, in ihrer Konzentration stark voneinander abweichende Liganden enthalten, die Konzentrationen der einzelnen Liganden mit nur einem einzigen Versuch zu bestimmen. Die Konzentration des Antikörper-Fragments in dem aus der Probe und dem Antikörper-Fragment bestehenden Gemisch und die Einwirkungsdauer, während der das Gemisch mit den Rezeptoren in Kontakt steht, werden dabei so auf die für die einzelnen Liganden zu überprüfenden Konzentrationsbereiche abgestimmt, dass nach dem Entfernen der nicht an einen Rezeptor gebundnen Bestandteile des Gemischs an allen Teststellen nur ein Teil der Bindungsstellen Rezeptoren an einen Liganden bindet, wenn dieser in dem zu untersuchenden Konzentrationsbereich in der Probe enthalten ist. Die Konzentration des zweiten Liganden kann mit Hilfe des Massenwirkungsgesetzes aus dem zweiten Messsignals und der bekannten Konzentration des Antikörper-Fragments in dem Gemisch berechnet werden. Die Konzentration des Antikörper-Fragments in dem Gemisch wird vorzugsweise so gewählt, dass sie einem Grenzwert der Konzentration des zweiten Liganden entspricht, aus dem sich eine Aussage, wie z.B. "gut/schlecht" oder "krank/gesund", ableiten lässt. Mit dem erfindungsgemäBen Verfahren können in einer Flusszelle oder dergleichen Messkammer nebeneinander Liganden-Konzentrationen in einem Bereich von g/l bis µg/l nachgewiesen werden.

Aufgrund der Monovalenz der Antikörper-Fragmente blockiert dann jedes gebundene Antikörper-Fragment genau eine einzige Bindungsstelle. Im Vergleich zu einem Verfahren, bei dem anstelle der monovalenten Antikörper-Fragmente bioder multivalente Moleküle verwendet werden, die jeweils an mehrere Bindungsstellen oder sogar an mehrere Liganden gleichzeitig binden können, ergibt sich dadurch eine deutlich verbesserte Messgenauigkeit.

Zweckmößigerweise stimmt das Antikörper-Fragment mit einem Fragment des zweiten Rezeptors überein. Das Antikörper-Fragment bindet dann bei einem Kontakt mit den zweiten Liganden sicher an die zweiten Bindungsstellen und blockiert diese.

Vorteilhaft ist, wenn das mindestens eine Antikörper-Fragment ein Fragment eines polyclonalen Antikörpers ist. Die Antikörper-Fragmente können dann beispielsweise aus dem Blut eines Tieres gewonnen werden.

Bei einer bevorzugten Ausgestaltung der Erfindung ist das mindestens eine Antikörper-Fragment ein Fragment eines monoclonalen Antikörpers. Die Antikörper-Fragmente sind dann kostengünstig In großen Mengen herstellbar.

Zweckmäßigerweise ist das mindestens eine Antikörper-Fragment ein Fab-Fragment, insbesondere ein Fab-Fragment der Moleküle lgG, lgA, lgM und/oder lgE. Diese Antikörper-Fragmente sind im Handel verfügbar. Bevorzugt werden Antikörper der Klasse lgG verwendet.

Bei einer vorteilhaften Ausführungsform der Erfindung wird ein für den ersten Liganden bindungsspezifischer, mit einem ersten Marker markierter Nachweisantikörper mit der ersten Teststelle in Kontakt gebracht, wobei danach nicht an einen Immobilisierten Rezeptor gebundene Marker von dem Substrat entfernt werden, und wobei danach das erste Messsignal in Abhängigkeit von der Konzentration des ersten Markers erzeugt wird. Die Konzentration des ersten Liganden kann also mit Hilfe eines Sandwich-Elisa gemessen werden.

Bei einer bevorzugten Ausgestaltung der Erfindung wird ein für die Liganden der zweiten Ligandenart bindungsspezifischer, mit einem zweiten Marker markierter Nachweisantikörper mit der zweiten Teststelle in Kontakt gebracht, wobei danach nicht an einen immobilisierten Rezeptor gebundene Marker von dem Substrat entfernt werden, und wobei dann das zweite Messsignal in Abhängigkeit von der Konzentration des zweiten Markers und der Konzentration des mindestens einen Antikörper-Fragments in dem Gemisch erzeugt wird. Es kann also auch die Konzentration des zweiten Liganden mit Hilfe eines Sandwich-Elisa gemessen werden.

Vorteilhaft ist, wenn der erste Marker und/oder zweite Marker ein Enzym ist (sind), wenn das Enzym während der Erfassung der Messsignale mit mindestens zwei Chemikalien In Kontakt gebracht wird, zwischen denen bei Anwesenheit des Enzyms eine chemische Redoxreaktion auftritt, und wenn das erste Messsignal und/oder das zweite Messsignal durch Messen eines Redoxpotentials erzeugt wird (werden). Dabei kann das Redoxpotential mit Hilfe eines ISFET gemessen werden.

Bei einer zweckmäßigen Ausgestaltung der Erfindung wird (werden) der erste Marker und/oder zweite Marker während der Erfassung der Messsignale mit einer Anregungsstrahlung bestrahlt, welche den (die) Marker zur Abgabe einer Lumineszenzstrahlung anregt, wobei das erste Messsignal und/oder das zweite Messsignal durch Messen der Lumineszenzstrahlung des betreffenden Markers erzeugt wird (werden). Dabei kann die Anregungsstrahlung mit Hilfe einer Lichtquelle, beispielsweise einer Leuchtdiode und/oder einer Xenon-Lampe erzeugt werden. Für den ersten und zweiten Marker wird vorzugsweise dieselbe Anregungsstrahlung verwendet. Es ist aber auch denkbar, dass der erste und zweite Marker unterschiedliche Farbstoffe, wie z.B. Cy3 und Cy5 sind, und dass diese Farbstoffe mit unterschiedlichen Wellenlängen angeregt werden.

Vorteilhaft ist, wenn die erste Teststelle und/oder zweite Testelle während der Erfassung der Messsignale mit einem Chemilumineszenzsubstrat in Kontakt gebracht wird (werden), in dem in Abhängigkeit von der Bindung der Liganden der ersten Ligandenart an den ersten Rezeptor und/oder in Abhängigkeit von der Bindung der Liganden der zweiten Ligandenart an den zweiten Rezeptor eine Lumineszenzstrahlung angeregt wird, und wenn das erste Messsignal und/oder das zweite Messsignal durch Messen der Lumineszenzstrahlung an der betreffenden Teststelle erzeugt wird (werden). Die Lumineszenzstrahlung wird dabei ohne eine Anregungsstrahlung auf chemischem Wege erzeugt.

Die vorstehend genannte Aufgabe wird bezüglich der Vorrichtung dadurch gelöst dass in der Messkammer mindestens ein nicht immobilisiertes monovolentes Antikörper-Fragment angeordnet ist, das für die zweite Bindungsstelle bindungsspezifisch ist und bei einem Kontakt mit der zu analysierenden Probe mit dieser ein Gemisch bildet, welches das Antikörper-Fragment in vorgegebener Konzentration enthält, und dass der zweite Sensor mit einer Auswerteeinrichtung verbunden ist, die zum Bestimmen der Konzentration des zweiten Liganden in der Probe aus dem zweiten Messsignal und der vorgegebenen Konzentration des Antikörper-Fragments in dem Gemisch ausgebildet ist.

Die Probe wird also während und/oder nach dem Eintritt in die Messkammer mit dem Antikörper-Fragment vermischt, so dass der zweite Ligand durch das Antikörper-Fragment teilweise weggefangen und die verfügbare Konzentration dieses Liganden dadurch reduziert wird. Wie bereits bei dem Verfahren erläutert wurde, können dadurch bei einer Probe, die mehrere, in ihrer Konzentration stark voneinander abweichende Liganden enthält, die Konzentrationen der einzelnen Liganden mit nur einem einzigen Versuch bestimmt werden.

Vorteilhaft ist, wenn das mindestens eine Antikörper-Fragment in gelförmiger, teigiger oder fester Form in der Messkammer stabilisiert ist, vorzugsweise derart, dass es an einer Begrenzungswand der Messkammer anhaftet. Die Vorrichtung ist dadurch besonders gut handhabbar. Die Stabilisierung des Antikörper-Fragments umfasst vorzugsweise mindestens ein nicht-reduzierendes Disaccharid und mindestens ein Protein oder Polypeptid der LEA-Klasse. Das nicht-reduzlerende Disaccharid kann aus einer Gruppe ausgewählt sein, die aus Trehaose (D-Glucopyranosyl-D-glucopyranose), Sucrose (β-D-Fructofuranosyl-α-D-glucopyranosid) sowie Derivaten davon ausgewählt sein kann. Eine derartige Stabilisierung ist in WO 2004/004455 A2 beschrieben. Der in stabilisierter Form vorliegende Kompetitor ist über längere Zeit haltbar.

Die vorstehend genannte Aufgabe wird bezüglich der Vorrichtung auch dadurch gelöst, dass die Vorrichtung eine Mischeinrichtung aufweist, die zum Vermischen der Probe mit mindestens einem für die zweite Bindungsstelle bindungsspezifischen monovalenten Antikörper-Fragment mit einer Zuführöffnung für die Probe und einem das Antikörper-Fragment enthaltenden Aufnahmeraum verbunden ist, dass die Mischeinrichtung eine Abgabeöffnung für das aus der Probe und dem Antikörper-Fragment gebildete Gemisch aufweist, die mit der Einlassöffnung der Messkammer verbunden ist, dass die Mischeinrichtung derart ausgestaltet ist, dass das Antikörper-Fragment in einer vorgegebenen Konzentration in dem Gemisch vorliegt, und dass der zweite Sensor mit einer Auswerteeinrichtung verbunden ist, die zum Bestimmen der Konzentration des zweiten Liganden in der Probe aus dem zweiten Messsignal und der Konzentration des Antikörper-Fragments in dem Gemisch ausgebildet ist.

Bei dieser Lösung wird die Probe also bereits vor dem Eintritt in die Messkammer mit Hilfe der Mischeinrichtung mit dem monovalenten Antikörper-Fragment vermischt. Dadurch wird eine besonders homogene Verteilung des monovalenten Antikörper-Fragments in der Probe ermöglicht Die Konzentration des zweiten Liganden kann dadurch mit großer Präzision gemessen werden.

Weitere vorteilhafte Ausgestaltungen der Erfindung sind in den Unteransprüchen beschrieben. Zweckmäßigerweise ist das mindestens einen Antikörper-Fragment in gelförmiger, teigiger oder fester Form in dem Aufhahmeraum stabilisiert, vorzugsweise derart, dass es an einer Begrenzungswand des Aufnahmeraums anhaftet, und dass der Aufnahmeraum zum Lösen des mindestens einen Antikörper-Fragments in der Probe als Durchflussmischkammer ausgebildet ist, über welche die Zuführöffnung für die Probe mit der Einlassöffnung der Messkammer verbunden ist. Die Vorrichtung ist dadurch auf einfache Weise handhabbar.

Bei einer bevorzugten Ausgestaltung der Erfindung weist die Durchflussmisckammer eine Mischerstruktur auf, die derart ausgestaltet ist, dass das Gemisch beim Durchfluss durch die Durchflussmischkammer vorzugsweise in abwechselnd zueinander entgegengesetzt verlaufende Richtungen abgelenkt wird, wobei die Mischerstruktur zwischen dem in gelförmiger, teigiger oder fester Form vorliegen mindestens einen Antikörper-Fragments und der Einlassöffnung der Messkammer angeordnet ist. Dabei kann der Mischer ein so genannter Möbiusmischer sein, der mit Methoden der Mikrosystemtechnik mit sehr kompakten Abmessungen hergestellt werden kann.

Bei einer zweckmäßigen Ausführungsform der Erfindung sind die Sensoren optische Sensoren, wobei der erste Rezeptor zur Detektion einer In Abhängigkeit von der Bindung der Liganden der ersten Ligandenart an den ersten Rezeptor erzeugten ersten Lumineszenzstrahlung vorzugsweise direkt auf dem ersten Sensor und/oder der zweite Rezeptor zur Detektion einer in Abhängigkeit von der Bindung der Liganden der zweiten Ligandenart an den zweiten Rezeptor erzeugten zweiten Lumineszenzstrahlung vorzugsweise direkt auf dem zweiten Sensor angeordnet Ist (sind). Die an den Rezeptoren erzeugte Lumineszenzstrahlung kann dann direkt ohne den Umweg über eine Sammellinse in den (die) Sensor(en) eingekoppelt werden.

Die Vorrichtung kann Bestandteil eines Kits nach einem der Ansprüche 22 bis 27 sein, der zusätzlich zu der der Vorrichtung
- einen mit einem Enzym oder dergleichen Marker markierten, für den ersten und/oder zweiten Liganden bindungsspezifischen Nachweisantikörper,
- mindestens zwei Chemikalien, zwischen denen bei einem Kontakt mit dem an dem Nachweisantikörper angeordneten Enzym eine chemische Redoxreaktion aurftritt, und/oder
- ein Chemilumineszenzsubstrat, in dem bei einem Kontakt mit dem an dem Nachweisantikörper angeordneten Enzym eine chemische Reaktion ausgelöst wird, bei der eine Lumineszenzstrahlung freigesetzt wird, und/oder
- eine Strahlungsquelle, die zur Aussendung der Lumineszenzstrahlung auf die erste Teststelle und/oder zweite angeordnet ist,
aufweisen kann. Der Marker, das Chemilumineszenzsubstrat und/oder die Chemikalien können mit einer geeigneten Zuführeinrichtung, wie z.B. einer Mikropumpe oder einer Pipette, der Messkammer zugeführt werden.

Nachfolgend Ist ein Ausführungsbeispiel der Erfindung anhand der Zeichnung näher erläutert. Es zeigen:
- Fig. 1: einen Längsschnitt durch ein erstes Ausführungsbeispiel einer Vorrichtung zum Bestimmen der Konzentrationen von in einer zu analysierenden Probe enthaltenen Liganden,
- Fig. 2: eine Teildarstellung einer Messkammer der Vorrichtung, die mit einer zu analysierenden Probe befüllt ist, wobei auf einer Wandung der Messkammer Immobilisierte Rezeptoren und in der Probe enthaltene Liganden und Antikörper-Fragmente schematisch angedeutet sind,
- Fig. 3: eine Teildarstellung der Messkammer nach dem Markieren von Rezeptor-Ligandenkomplexen,

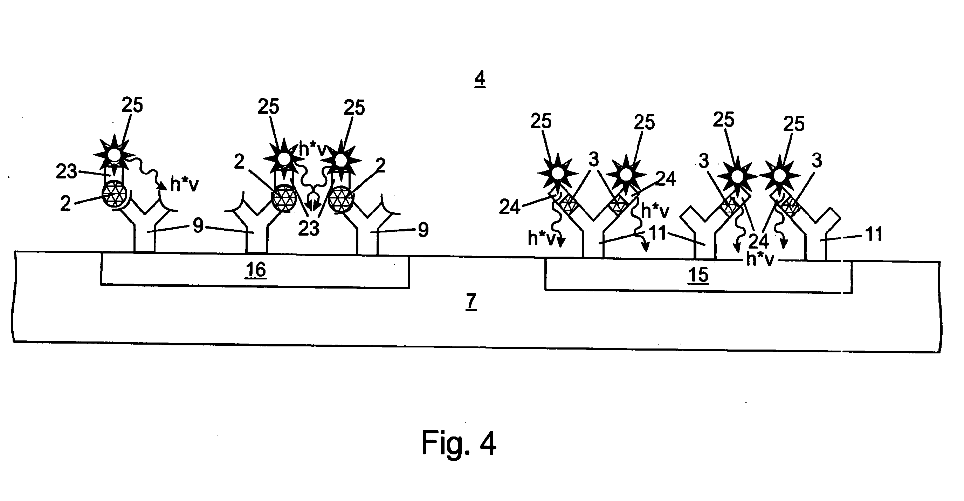

- Fig.4: eine Teildarstellung der Messkammer nach dem Einbringen eines Chemi-Lumineszenzsubstrats in die Messkammer, und
- Fig. 5: einen Längsschnitt durch ein zweites Ausführungsbeispiel einer Vorrichtung zum Bestimmen der Konzentrationen von In einer zu analysierenden Probe enthaltenen Liganden.

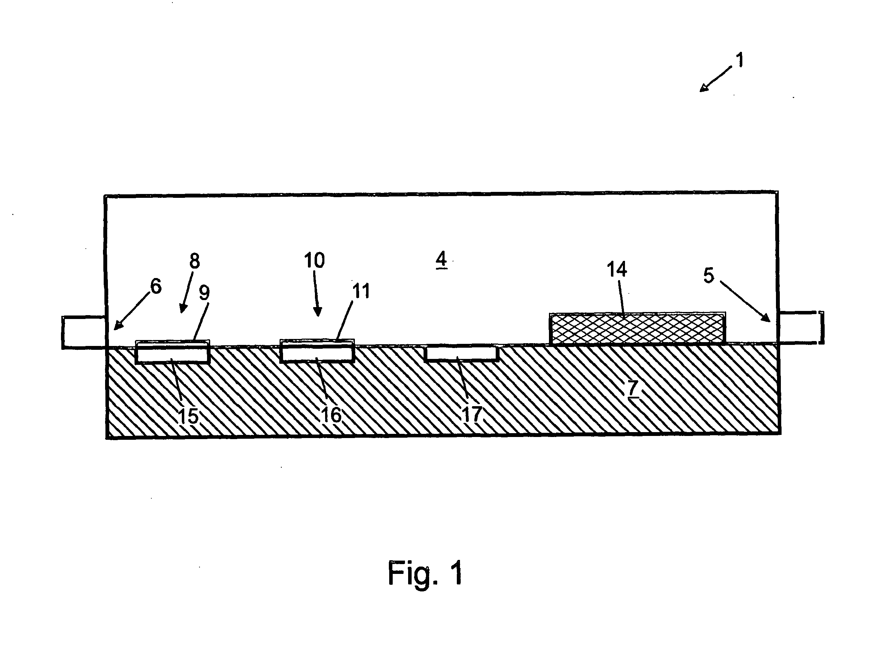

Eine in Fig. 1 im Ganzen 1 mit bezeichnete Vorrichtung zum Bestimmen der Konzentrationen von mindestens zwei Liganden 2, 3 in einer zu analysierenden Probe weist eine als Flusszelle ausgebildete Messkammer 4 auf, die eine Einlassöffnung 5 und eine Auslassöffnung 6 hat. Eine Wandung der Messkammer 4 ist durch ein Halbleiter-Substrat 7 gebildet, auf dem an einer ersten Teststelle 8 mindestens ein erster Rezeptor 9 ersten Rezeptorart und an einer seitlich davon beabstandeten zweiten Teststelle 10 mindestens ein zweiter Rezeptor 11 einer zweiten Rezeptorart immobilisiert sind. Der erste Rezeptor 9 ist für einen ersten, in der zu analysierenden Probe enthaltenen Liganden 2 und der zweite Rezeptor 11 für einen zweiten, In der Probe enthaltenen Liganden 3 bindungsspezifisch. Der erste Ligand 2 kann beispielsweise lgE und der zweite Ligand 3 CRP (C-reaktives Protein) sein. Der zweite Ligand 3 weist eine wesentlich größere Konzentration In der Probe auf als der erste Ligand 2. Der zweite Rezeptor 11 kann beispielsweise ein Antikörper der lgG Klasse sein.

In der Messkammer 4 ist ferner mindestens ein monovalentes Antikörper-Fragment 14 der zweiten Rezeptorart angeordnet, das nicht immobilisiert ist und in lyophiliserter Form vorliegt. Das Antikörper-Fragment 14 haftet an einer Begrenzungswand der Messkammer 4 an. Bei einem Kontakt mit der zu analysierenden wässrigen Probe löst sich das Antikörper-Fragment 14 in der Probe. Das Antikörper-Fragment 14 kann beispielsweise ein Anti-CRP Fab Fragment sein. Dieses kann durch Papainverdau der bereits erwähnten Antikörper der lgG Klasse gewonnen werden. Dabei werden die Antikörper mit dem Immunopure Fab Preparation Kit von Pierce (Product #44885) gemäß der Anleitung des Herstellers verdaut.

An der ersten Teststelle 8 ist direkt unter dem ersten Rezeptor 9 ein erster optischer Sensor 15 und an der zweiten Teststelle 10 direkt unter dem zweiten Rezeptor 11 ein zweiter optischer Sensor 16 in das Halbleiter-Substrat 23 integriert. Zusätzlich ist ein dritter optischer Sensor 17 in der Wandung der Messkammer 4 angeordnet, der nicht mit einem Rezeptor bedeckt ist und als Referenzwertgeber dient. Die Sensoren 15, 16, 17 können beispielsweise Photodioden sein.

Zum Bestimmen der Konzentrationen der Liganden 2, 3 wird zunächst die Probe - ein Serum mit einem Gehalt von 10 µg/ml CRP - durch die Einlassöffnung 5 hindurch derart in die Messkammer 4 eingefüllt, dass das gesamte Volumen der Messkammer 4 mit der Probe befüllt ist. Sobald die Antikörper-Fragmente 14 mit der Probe in Kontakt kommen, lösen sie sich in dieser. Dabei wird das in dem Serum enthaltene C-raktive Protein mit der 2,5-fachen molaren Menge an Anti-CRP Fab Fragmenten versetzt und gemischt. Der Lösungsvorgang kann ggf durch Einringen von mechanischer Energie In die Messkammer 4 beschleunigt werden. Die Menge der Antikörper-Fragmente 14 ist derart auf das Volumen der Messkammer 4 abgestimmt, dass das aus der Probe und den Antikörper-Fragmenten 14 gebildete Gemisch die Antikörper-Fragmente 14 In einer vorgegebenen, einem zu messenden Grenzwert entsprechenden Konzentration enthält, wenn diese vollständig und homogen in der Probe gelöst sind.

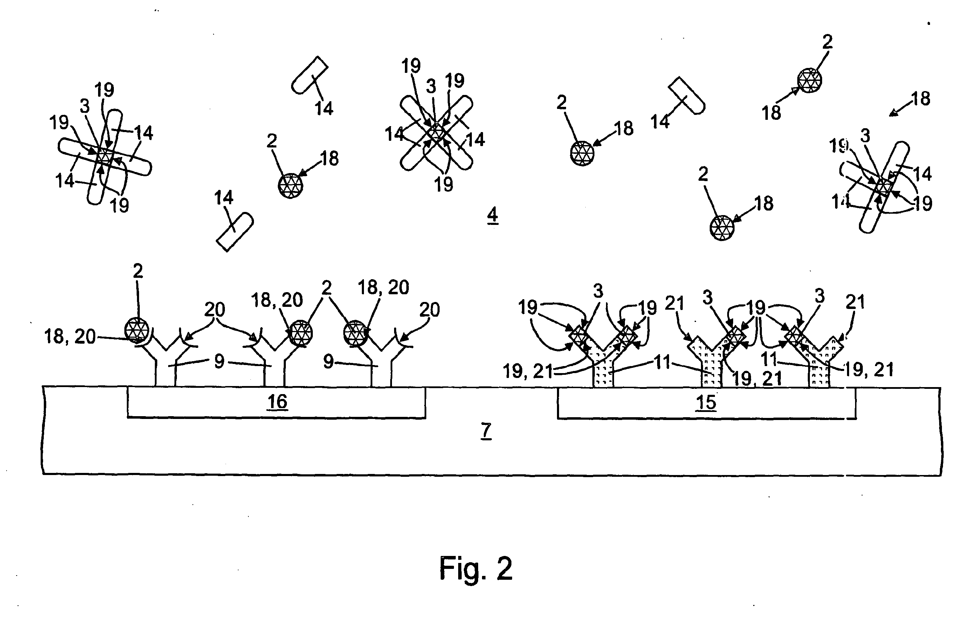

Nachdem die Messkammer 4 mit der Probe befüllt wurde, wird eine vorgegebene erste Zeitdauer abgewartet, während der sich die Antikörper-Fragmente 14 in der Probe lösen können. Wie in Fig. 2 erkennbar ist, wird die erste Zeitdauer so gewählt, dass die in der Probe enthaltenen ersten Liganden 2 die Möglichkeit haben, jeweils an einen ersten Rezeptor 9 und die in der Probe enthaltenen zweiten Liganden 3 die Möglichkeit haben, an einen zweiten Rezeptor 11 und/oder ein monovalentes Antikörper-Fragment 14 zu binden. Deutlich ist erkennbar, dass jeweils nur ein Teil der an den einzelnen Teststellen 10, 11 vorhanden freien Bindungsstellen der Rezeptoren 9, 11 an einen Liganden 2, 3 bindet.

In Fig. 2 ist ferner erkennbar, dass die ersten Liganden 2 jeweils eine erste Bindungsstelle 18 und die zweiten Liganden 3 jeweils mehrere zweite Bindungsstellen 19 aufweisen. Die ersten Rezeptoren 9 haben jeweils mehrere dritte Bindungsstellen 20, die bei einem Kontakt mit einer ersten Bindungsstelle 18 spezifisch an diese binden können. In entsprechender Weise haben die zweiten Rezeptoren 11 jeweils mehrere vierte Bindungsstellen 21, die bei einem Kontakt mit einer zweiten Bindungsstelle 19 spezifisch an diese binden können. Jedes monovalente Antikörper-Fragment 14 hat jeweils genau eine Bindungsstelle, die für die zweiten Bindungsstellen 19, nicht jedoch für die ersten Bindungsstellen 18 bindungsspezifisch ist.

Nachdem die vorgegebene erste Zeitdauer, die beispielsweise eine Stunde betragen kann, abgelaufen ist, wird über eine Zuführöffnung 22, die Einlassöffnung 5 und die Auslassöffnung 6 eine Spülflüssigkeit durch die Messkammer 4 hindurchgeleitet, welche die nicht an einen Rezeptor 9, 11 gebundenen Bestandteile des Gemischs aus der Messkammer 4 entfernt. Die Spülflüssigkeit kann beispielsweise PBS sein.

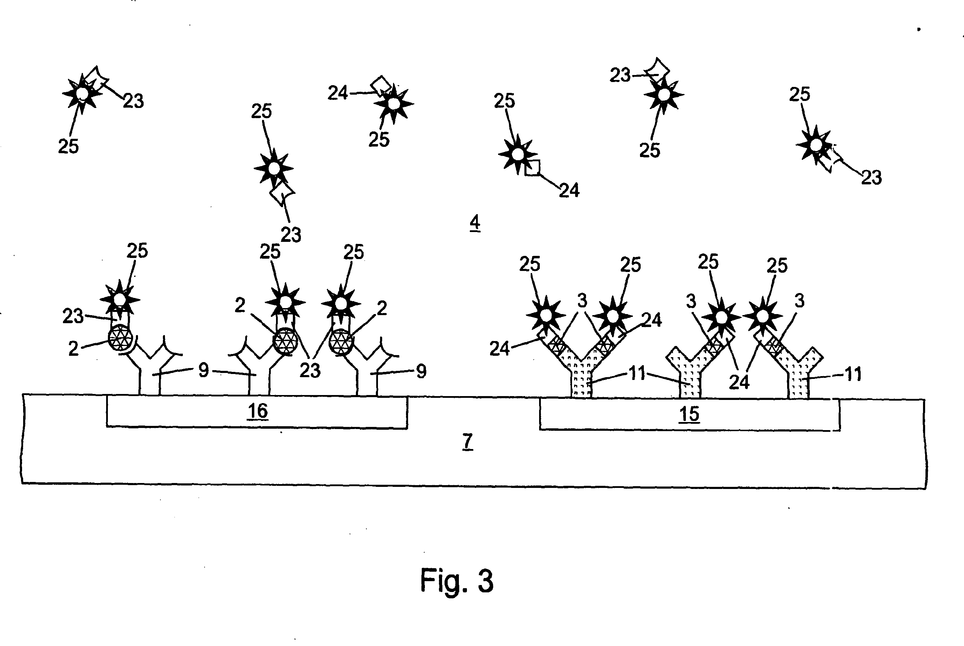

Danach wird eine Lösung in die Messkammer 4 eingebracht, die Nachweisantikörper 23, 24 enthält Ein erster Nachweisantikörper 23 ist für die ersten Bindungsstellen 18 und ein zweiter Nachweisantikörper 24 für die zweiten Bindungsstellen 19 beindungsspezifisch. Die Nachweisantikörper 23, 24 sind jeweils mit einem Enzym 25, wie z.B. HRP, markiert Nachdem der Nachweisantikörper 23, 24 in die Messkammer 4 eingebracht wurde, wird eine vorgegebene zweite Zeitdauer abgewartet, die so gewählt ist, dass nahezu alle an den ersten Rezeptor 9 gebundenen ersten Liganden 2 jeweils an einen ersten Nachweisantikörper 23 und nahezu alle an den zweiten Rezeptor 11 gebundenen zweiten Liganden 3 jeweils an einen zweiten Nachweisantikörper 24 binden und dadurch indirekt mit dem Enzym 25 markiert werden (Fig. 3). Die zweite Zeitdauer kann beispielsweise 15 Minuten betragen.

Nachdem die vorgegebene zweite Zeitdauer abgelaufen ist, wird erneut Spülflüssigkeit durch die Messkammer 4 hindurchgeleitet, um nicht an einen Liganden 2, 3 gebundene Nachweisantikörper 23, 24 aus der Messkammer 4 zu entfernen.

Danach wird über die Einlassöffnung 5 ein Chemi-Lumineszenzsubstrat in die Messkammer 4 eingeleitet, das Wasserstoffperoxid und einen Chemiluminophor, wie z.B. Luminol, enthält. Ein derartiges Chemi-Lumineszenzsubstrat ist im Handel unter der Bezeichnung ECL-Lösung verfügbar. Wenn das Enzym 25 mit dem Wasserstoffperoxid in Kontakt gerät, werden freie Sauerstoffradikale von dem Wasserstoffperoxid abgespalten, wodurch der Chemiluminophor unter Abgabe von Lumineszenzstrahlung chemisch zersetzt wird. Die Lumineszenzstrahlung wird also sowohl In Abhängigkeit von der Bindung der ersten Bindungsstellen 18 an die dritten Bindungsstellen 20 als auch in Abhängigkeit von der Bindung der zweiten Bindungsstellen 19 an die vierten Bindungsstellen 21 erzeugt.

Die optischen Sensoren 15, 16 sind für die Lumineszenzstrahlung empfindlich und derart relativ zu den Rezeptoren 9, 11 angeordnet, dass sie jeweils nur die an der ihnen zugeordneten Teststelle 8 (10) erzeugte Lumineszenzstrahlung detektieren, nicht jedoch die Lumineszenzstrahlung, die an der jeweils anderen Teststelle 10 (8) erzeugt wird.

Mit den Sensoren 15, 16 ist eine in der Zeichnung nicht näher dargestellte Auswerteeinrichtung verbunden, die in Abhängigkeit von dem Messsignal des ersten Sensors 15 die Konzentrationen der ersten Liganden 2 in der Probe und in Abhängigkeit von dem Messsignal des zweiten Sensors 16 und der bekannten Konzentration der monovalenten Antikörper-Fragmente 14 in dem Gemisch mit Hilfe des Massenwirkungsgesetzes die Konzentration der zweiten Liganden 3 in der Probe bestimmt. Das Messsignal ist proportional zur Konzentration der Liganden 2, 3 im Serum. Die Auswerteeinrichtung kann als elektrische Schaltung in das Substrat 7 bzw. die Wandung der Messkammer 4 integriert sein.

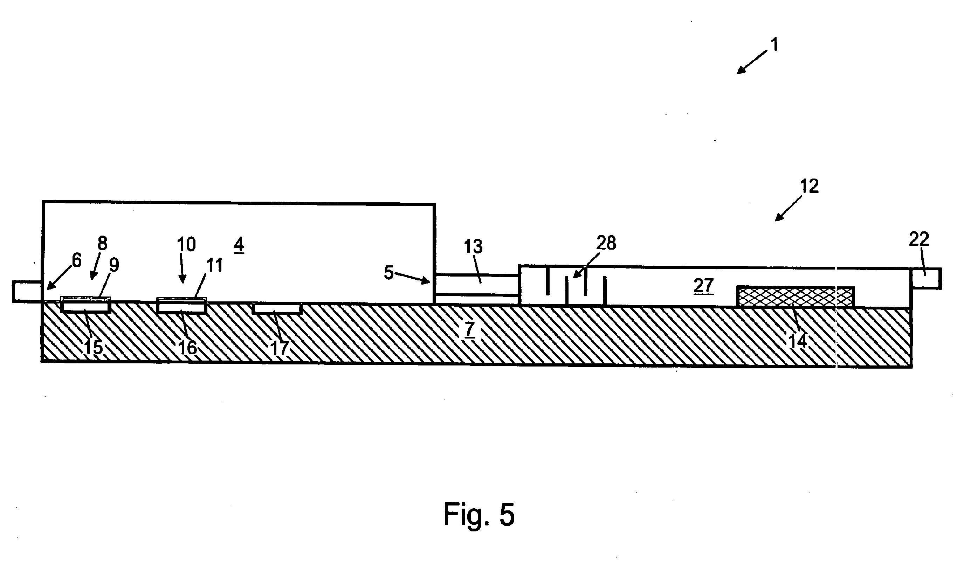

Bei dem in Fig. 5 gezeigten Ausführungsbeispiel weist die Vorrichtung 1 eine der Messkammer 4 vorgeschaltete Mischeinrichtung 26 auf, welche die Einlassöffnung 5 mit der Zuführöffnung 22 verbindet. Zwischen der Zuführöffnung 22 und der Einlassöffnung 5 Ist ein Aufnahmeraum 27 angeordnet, der die monovalenten Antikörper-Fragmente 14 In stabilisierter Form enthält. Die Mischeinrichtung 26 dient dazu, die Probe mit den Antikörper-Fragmenten 14 homogen zu vermischen. Dazu wird die Probe beispielsweise mit Hilfe einer Pipette oder einer Pumpe durch die Zuführöffnung 22 hindurch in den Aufnahmeraum 27 eingeleitet. In dem Aufnahmeraum 27 sind die Antikörper-Fragmente 14 derart relativ zu der Zuführöffnung 22 angeordnet, dass die Probe während und/oder nach dem Einleiten in den Aufnahmeraum 27 mit den Antikörper-Fragmenten 14 in Kontakt gerät.

Die Mischeinrichtung 26 ist derart ausgestaltet, dass die Antikörper-Fragmente 14 in der Mischkammer 4 in einer vorgegebenen, einem zu messenden Grenzwert entsprechenden Konzentration in dem Gemisch vorliegen. Dies wird dadurch erreicht, dass die Messkammer 4 und die Mischeinrichtung 26 ein vorbestimmtes Volumen ausweisen, und dass die Menge der In dem Aufnahmeraum 27 stabilisierten die Antikörper-Fragmente 14 so gewählt ist, dass sich die gewünschte Konzentration einstellt, wenn die die Antikörper-Fragmente 14 mit der Probe zu einem Gemisch vermischt werden, welches das vorbestimmte Volumen ausweist.

In Strömungsrichtung hinter dem Aufnahmeraum 27 weist die Mischeinrichtung 26. eine als Möbiusmischer ausgestaltete Mischerstruktur 12 auf, die mit ihrem einen Ende mit dem Aufnahmeraum 27 und mit ihrem anderen, eine Abgabeöffnung für das aus der Probe und den Antikörper-Fragmenten 14 gebildete Gemisch aufweisenden Ende über einen Kanal 13 mit der Elnlassöffnung 5 der Messkammer 4 verbunden ist.

Der Aufbau der Messkammer 4 entspricht im Wesentlichen dem in Fig. 1, jedoch mit dem Unterschied, dass in der Messkammer keine Antikörper-Fragmente 14 deponiert sind. Insoweit wird auf die dortige Beschreibung verwiesen.

## Patentansprüche

1. Verfahren zum Bestimmen der Konzentrationen von Liganden (2; 3) in einer zu analysierenden Probe, wobei die Probe mindestens einen Ligand (2) einer ersten Ligandenart aufweist, der zumindest eine erste Bindungsstelle (18) hat, für die Rezeptoren (9) einer ersten Rezeptorart bindungsspezifisch sind, wobei die Probe mindestens einen Ligand (3) einer zweiten Ligandenart aufweist, der zumindest eine zweite Bindungsstelle (19) hat, für die Rezeptoren (11) einer zweiten Rezeptorart bindungsspezifisch sind, wobei mindestens ein erster Rezeptor (9) der ersten Rezeptorart an wenigstens einer ersten Teststelle (8) und zumindest ein zweiter Rezeptor (11) der zweiten Rezeptorart an wenigstens einer zweiten Teststelle (10) auf einem Substrat (7) immobilisiert werden, wobei mindestens ein Antikörper-Fragment (14) bereitgestellt wird, das für die zweite Bindungsstelle (19) bindungsspezifisch ist, wobei das mindestens eine Antikörper-Fragment (14) derart mit der Probe vermischt wird, dass es in einer vorgegebenen Konzentration in dem so erhaltenen Gemisch vorliegt, wobei das Gemisch danach zum Binden der Liganden (2, 3) an die Rezeptoren (9, 11) derart mit dem Substrat (7) in Kontakt gebracht wird, dass mindestens ein Ligand (2) der ersten Ligandenart an den mindestens einen ersten Rezeptor (9) und wenigstens ein Ligand (3) der zweiten Ligandenart an den zumindest einen zweiten Rezeptor (11) binden können, wobei Bestandteile des Gemischs, die nicht an einen immobilisierten Rezeptor (9, 11) gebunden sind, von dem Substrat (7) entfernt werden, wobei ein von der Konzentration des an den ersten Rezeptor (9) gebundenen Liganden (2) abhängiges erstes Messsignal und ein von der Konzentration des an den zweiten Rezeptor (1.1) gebundenen Liganden (3) abhängiges zweites Messsignal erzeugt werden, wobei mit Hilfe des ersten Messsignals die Konzentration des Liganden (2) der ersten Ligandenart und die Konzentration der Liganden (3) der zweiten Ligandenart mit Hilfe des zweiten Messsignals und der Konzentration des mindestens einen Antikörper-Fragments (14) in dem Gemisch bestimmt werden, **dadurch gekennzeichnet, dass** das mindestens eine Antikörper-Fragment (14) monovalent ist.

2. Verfahren nach Anspruch 1, **dadurch gekennzeichnet, dass** zumindest der Ligand (3) der zweiten Ligandenart wenigstens zwei Bindungsstellen (19) für die Antikörper-Fragmente (14) hat.

3. Verfahren nach Anspruch 1 oder 2, **dadurch gekennzeichnet**, **dadurch gekennzeichnet, dass** das Antikörper-Fragment (14) mit einem Fragment des zweiten Rezeptors (11) übereinstimmt.

4. Verfahren nach einem der Ansprüche 1 bis 3, **dadurch gekennzeichnet, dass** das mindestens eine Antikörper-Fragment (14) ein Fragment eines polyclonalen Antikörpers ist.

5. Verfahren nach einem der Ansprüche 1 bis 4, **dadurch gekennzeichnet, dass** das mindestens eine Antikörper-Fragment (14) ein Fragment eines monoclonalen Antikörpers ist.

6. Verfahren nach einem der Ansprüche 1 bis 5, **dadurch gekennzeichnet, dass** das mindestens eine Antikörper-Fragment (14) ein Fab-Fragment ist, insbesondere ein Fab-Fragment der Moleküle IgG, IgA, IgM und/oder IgE.

7. Verfahren nach einem der Ansprüche 1 bis 6, **dadurch gekennzeichnet, dass** ein für die Liganden (2) der ersten Ligandenart bindungsspezifischer, mit einem ersten Marker markierter erster Nachweisanfikörper (23) mit der ersten Teststelle (8) in Kontakt gebracht wird, dass danach nicht an einen immobilisierten Rezeptor (9, 11) gebundene Marker von dem Substrat (7) entfernt werden, und dass dann das erste Messsignal in Abhängigkeit von der Konzentration des ersten Markers erzeugt wird.

8. Verfahren nach einem der Ansprüche 1 bis 7, **dadurch gekennzeichnet, dass** ein für die Liganden (3) der zweiten Ligandenart bindungsspezifischer, mit einem zweiten Marker markierter zweiter Nachweisantikörper (24) mit der zweiten Teststelle (10) in Kontakt gebracht wird, dass danach nicht an einen immobilisierten Rezeptor (9, 11) gebundene Marker von dem Substrat (7) entfernt werden, und dass dann das zweite Messsignal in Abhängigkeit von der Konzentration des zweiten Markers und der Konzentration des mindestens einen Antikörper-Fragments (14) in dem Gemisch erzeugt wird.

9. Verfahren nach einem der Ansprüche 1 bis 8, **dadurch gekennzeichnet, dass** der erste Marker und/oder zweite Marker ein Enzym ist (sind), dass das Enzym während der Erfassung der Messsignale mit mindestens zwei Chemikalien in Kontakt gebracht wird, zwischen denen bei Anwesenheit des Enzyms eine chemische Redoxreaktion auftritt, und dass das erste Messsignal und/oder das zweite Messsignal durch Messen eines Redoxpotentials erzeugt wird (werden).

10. Verfahren nach einem der Ansprüche 1 bis 9, **dadurch gekennzeichnet, dass** der erste Marker und/oder zweite Marker während der Erfassung der Messsignale mit einer Anregungsstrahlung bestrahlt wird (werden), welche den (die) Marker zur Abgabe einer Lumineszenzstrahlung anregt, und dass das erste Messsignal und/oder das zweite Messsignal durch Messen der Lumineszenzstrahlung des betreffenden Markers erzeugt wird (werden).

11. Verfahren nach einem der Ansprüche 1 bis 10, **dadurch gekennzeichnet, dass** die erste Teststelle (8) und/oder zweite Testelle (10) während der Erfassung der Messsignale mit einem Chemi-Lumineszenzsubstrat in Kontakt gebracht wird (werden), in dem in Abhängigkeit von der Bindung der Liganden (2) der ersten Ligandenart an den ersten Rezeptor (9) und/oder in Abhängigkeit von der Bindung der Liganden (3) der zweiten Ligandenart an den zweiten Rezeptor (11) eine Lumineszenzstrahlung angeregt wird, und dass das erste Messsignal und/oder das zweite Messsignal durch Messen der Lumineszenzstrahlung an der betreffenden Teststelle (8, 10) erzeugt wird (werden).

12. Vorrichtung (1) zum Bestimmen der Konzentrationen von Liganden (2, 3) unterschiedlicher Ligandenarten in einer zu analysierenden Probe, mit einer zumindest eine Einlassöffnung (5) und eine Auslassöffnung (6) aufweisenden Messkammer (4), die ein Substrat (7) hat, auf dem an einer ersten Teststelle (8) ein erster Rezeptor (9) einer ersten Rezeptorart und an einer zweiten Teststelle (10) ein zweiter Rezeptor (11) einer zweiten Rezeptorart immobilisiert sind, wobei die erste Rezeptorart für mindestens eine erste Bindungsstelle (18) der Liganden (2) der ersten Ligandenart für und die zweite Rezeptorart (11) für mindestens eine zweite Bindungsstelle (19) der Liganden (3) der zweiten Ligandenart bindungsspezifisch sind, wobei an der ersten Teststelle (8) ein erster Sensor (15) zum Erfassen eines von der Konzentration des an den ersten Rezeptor (9) gebundenen Liganden (2) abhängigen ersten Messsignals und der zweiten Teststelle (10) ein zweiter Sensor (16) zum Erfassen eines von der Konzentration des an den zweiten Rezeptor (11) gebundenen Liganden (3) abhängigen zweiten Messsignals zugeordnet sind, **dadurch gekennzeichnet, dass** in der Messkammer (4) mindestens ein nicht immobilisiertes monovalentes Antikörper-Fragment (14) angeordnet ist, das für die zweite Bindungsstelle (19) blndungsspezifisch ist und bei einem Kontakt mit der zu analysierenden Probe mit dieser ein Gemisch bildet, welches das Antikörper-Fragment (14) in vorgegebener Konzentration enthält, und dass der zweite Sensor (16) mit einer Auswerteeinrichtung verbunden ist, die zum Bestimmen der Konzentration des zweiten Liganden (3) in der Probe aus dem zweiten Messsignal und der vorgegebenen Konzentration des Antikörper-Fragments (14) in dem Gemisch ausgebildet ist.

13. Vorrichtung nach Anspruch 12, **dadurch gekennzeichnet, dass** das Antikörper-Fragment (14) in gelförmiger, teigiger oder fester Form in der Messkammer (4) stabilisiert ist, vorzugsweise derart, dass es an einer Begrenzungswand der Messkammer (4) anhaftet.

14. Vorrichtung (1) nach dem Oberbegriff von Anspruch 12, **dadurch gekennzeichnet, dass** die Vorrichtung eine Mischeinrichtung (26) aufweist, die zum Vermischen der Probe mit mindestens einem für die zweite Bindungsstelle (19) bindungsspezifischen monovalenten Antikörper-Fragment (14) mit einer Zuführöffnung (22) für die Probe und einem das Antikörper-Fragment (14) enthaltenden Aufnahmeraum (27) verbunden ist, dass die Mischeinrichtung (26) eine Abgabeöffnung für das aus der Probe und dem Antikörper-Fragment (14) gebildete Gemisch aufweist, die mit der Einlassöffnung (5) der Messkammer (4) verbunden ist, dass die Mischeinrichtung (26) derart ausgestaltet ist, dass das Antikörper-Fragment (14) in einer vorgegebenen Konzentration in dem Gemisch vorliegt, und dass der zweite Sensor (16) mit einer Auswerteeinrichtung verbunden ist, die zum Bestimmen der Konzentration des zweiten Liganden (3) in der Probe aus dem zweiten Messsignal und der Konzentration des Antikörper-Fragments (14) in dem Gemisch ausgebildet ist.

15. Vorrichtung (1) nach einem der Ansprüche 12 bis 14, **dadurch gekennzeichnet, dass** das Antikörper-Fragment (14) mit einem Fragment des zweiten Rezeptors übereinstimmt.

16. Vorrichtung (1) nach einem der Ansprüche 12 bis 15, **dadurch gekennzeichnet, dass** das mindestens eine Antikörper-Fragment (14) ein Fragment eines polyclonalen Antikörpers ist.

17. Vorrichtung (1) nach einem der Ansprüche 12 bis 16, **dadurch gekennzeichnet, dass** das mindestens eine Antikörper-Fragment (14) ein Fragment eines monoclonalen Antikörpers ist.

18. Vorrichtung (1) nach einem der Ansprüche 12 bis 17, **dadurch gekennzeichnet, dass** das mindestens eine Antikörper-Fragment (14) ein Fab-Fragment ist, insbesondere ein Fab-Fragment der Moleküle IgA, IgM und/oder IgE.

19. Vorrichtung (1) nach einem der Ansprüche 14 bis 18, **dadurch gekennzeichnet, dass** das Antikörper-Fragment (14) in getförmiger, teigiger oder fester Form in dem Aufnahmeraum (27) stabilisiert ist, vorzugsweise derart, dass es an einer Begrenzungswand des Aufnahmeraums (27) anhaftet, und dass der Aufnahmeraum (27) zum Lösen des Antikörper-Fragments (14) in der Probe als Durchflussmischkammer ausgebildet ist, über welche die Zuführöffnung (22) für die Probe mit der Einlassöffnung (5) der Messkammer (4) verbunden ist.

20. Vorrichtung (1) nach einem der Ansprüche 14 bis 19, **dadurch gekennzeichnet, dass** die Durchflussmischkammer eine Mischerstruktur (12) aufweist, die derart ausgestaltet ist, dass das Gemisch beim Durchfluss durch die Durchflussmischkammer vorzugsweise in abwechselnd zueinander entgegengesetzt verlaufende Richtungen abgelenkt wird, und dass die Mischerstruktur (12) zwischen dem in gelförmiger, teigiger oder fester Form vorliegenden Antikörper-Fragment (14) und der Einlassöffnung (5) der Messkammer angeordnet ist.

21. Vorrichtung (1) nach einem der Ansprüche 12 bis 20, **dadurch gekennzeichnet, dass** die Sensoren (15, 16) optische Sensoren sind, und dass der erste Rezeptor (9) zur Detektion einer in Abhängigkeit von der Bindung der Liganden (2) der ersten Ligandenart an den ersten Rezeptor (9) erzeugten ersten Lumineszenzstrahlung vorzugsweise direkt auf dem ersten Sensor (15) und/oder der zweite Rezeptor (11) zur Detektion einer in Abhängigkeit von der Bindung der Liganden (3) der zweiten Ligandenart an den zweiten Rezeptor (11) erzeugten zweiten Lumineszenzstrahlung vorzugsweise direkt auf dem zweiten Sensor (16) angeordnet ist (sind).

22. Kit zur Durchführung des Verfahrens nach einem der Ansprüche 1 bis 11, umfassend
- eine Vorrichtung (1) nach einem der Ansprüche 12 bis 21, bei welcher der erste Sensor (15) ein Sensor zur Messung eines Redoxpotentials ausgebildet ist,
- einen für Liganden (2) der ersten Ligandenart bindungsspezifischen, mit einem Enzym markierten Nachweisantikörper (23) und
- mindestens zwei Chemikalien, zwischen denen bei einem Kontakt mit dem Enzym eine chemische Redoxreaktion auftritt.

23. Kit zur Durchführung des Verfahrens nach einem der Ansprüche 1 bis 11, umfassend
- eine Vorrichtung (1) nach einem der Ansprüche 12 bis 21, bei welcher der zweite Sensor (16) ein Sensor zur Messung eines Redoxpotentials ausgebildet ist,
- einen für Liganden (3) der zweiten Ligandenart bindungsspezifischen, mit einem Enzym markierten Nachweisantikörper (24) und
- mindestens zwei Chemikalien, zwischen denen bei einem Kontakt mit dem Enzym eine chemische Redoxreaktion auftritt.

24. Kit zur Durchführung des Verfahrens nach einem der Ansprüche 1 bis 11, insbesondere nach Anspruch 23, umfassend
- eine Vorrichtung (1) nach einem der Ansprüche 12 bis 21,
- einen für Liganden der ersten Ligandenart (2) bindungsspezifischen, mit einem Enzym markierten Nachweisantikörper und
- ein Chemi-Lumineszenzsubstrat, in dem bei einem Kontakt mit dem Enzym eine chemische Reaktion ausgelöst wird, bei der eine Lumineszenzstrahlung freigesetzt wird, für die der erste Sensor (15) empfindlich ist.

25. Kit zur Durchführung des Verfahrens nach einem der Ansprüche 1 bis 11, insbesondere nach Anspruch 23, umfassend
- eine Vorrichtung (1) nach einem der Ansprüche 12 bis 21,
- einen für Liganden (3) der zweiten Ligandenart bindungsspezifischen, mit einem Enzym markierten Nachweisantikörper (24) und
- ein Chemi-Lumineszenzsubstrat, in dem bei einem Kontakt mit dem Enzym eine chemische Reaktion ausgelöst wird, bei der eine Lumineszenzstrahlung freigesetzt wird, für die der zweite Sensor (16) empfindlich ist.

26. Kit zur Durchführung des Verfahrens nach einem der Ansprüche 1 bis 11, insbesondere nach Anspruch 23 oder 25, umfassend
- einen für Liganden der ersten Ligandenart bindungsspezifischen Nachweisantikörper (23), der mit einem Marker markiert ist, der durch Bestrahlen mit einer Anregungsstrahlung zur Abgabe einer Lumineszenzstrahlung anregbar ist,
- eine Vorrichtung (1) nach einem der Ansprüche 12 bis 20, bei welcher der erste Sensor (15) für die Lumineszenzstrahlung empfindlich und für die Anregungsstrahlung unempfindlich ist, und
- eine Strahlungsquelle, die zur Aussendung der Anregungsstrahlung auf die erste Teststelle (8) angeordnet ist.

27. Kit zur Durchführung des Verfahrens nach einem der Ansprüche 1 bis 11, insbesondere nach Anspruch 22 oder 24, umfassend
- einen für Liganden (2) der ersten Ligandenart bindungsspezifischen Nachweisontikörper (23), der mit einem Marker markiert ist, der durch Bestrahlen mit einer Anregungsstrahlung zur Abgabe einer Lumineszenzstrahlung anregbar ist,
- eine Vorrichtung (1) nach einem der Ansprüche 12 bis 20, bei welcher der zweite Sensor (16) für die Lumineszenzstrahlung empfindlich und für die Anregungsstrahlung unempfindlich ist, und
- eine Strahlungsquelle, die zur Aussendung der Anregungsstrahlung auf die zweite Teststelle (10) angeordnet ist.

## Claims

1. Method for determining the concentrations of ligands (2, 3) in a sample to be analysed, wherein the sample has at least one ligand (2) of a first type of ligand that has at least one first binding site (18) which receptors (9) of a first type of receptor specifically bind to, wherein the sample has at least one ligand (3) of a second type of ligand that has at least one second binding site (19) which receptors (11) of a second type of receptor specifically bind to, wherein at least one first receptor (9) of the first type of receptor is immobilized to at least one first test site (8) and at least one second receptor (11) of the second type of receptor is immobilized to at least one second test site (10) on a substrate (7), wherein at least one antibody fragment (14) is provided which specifically binds to the second binding site (19), wherein the at least one antibody fragment (14) is mixed with the sample in such a way that the mixture thus obtained contains a predetermined concentration of said fragment, wherein, for the ligands (2, 3) to bind to the receptors (9, 11), the mixture is then contacted with the substrate (7) in such a way that at least one ligand (2) of the first type of ligand can bind to the at least one first receptor (9) and at least one ligand (3) of the second type of ligand can bind to the at least one second receptor (11), wherein components of the mixture that are not bound to an immobilized receptor (9, 11) are removed from the substrate (7), wherein a first measured signal is generated as a function of the concentration of the ligand (2) bound to the first receptor (9) and a second measured signal is generated as a function of the concentration of the ligand (3) bound to the second receptor (11), wherein the concentration of the ligand (2) of the first type of ligand is determined with the aid of the first measured signal and the concentration of the ligand (3) of the second type of ligand is determined with the aid of the second measured signal and the concentration of the at least one antibody fragment (14) in the mixture, **characterized in that** the at least one antibody fragment (14) is monovalent.

2. Method according to Claim 1, **characterized in that** at least the ligand (3) of the second type of ligand has at least two binding sites (19) for the antibody fragments (14).

3. Method according to Claim 1 or 2, **characterized in that** the antibody fragment (14) corresponds to a fragment of the second receptor (11).

4. Method according to any of Claims 1 to 3, **characterized in that** the at least one antibody fragment (14) is a fragment of a polyclonal antibody.

5. Method according to any of Claims 1 to 4, **characterized in that** the at least one antibody fragment (14) is a fragment of a monoclonal antibody.

6. Method according to any of Claims 1 to 5, **characterized in that** the at least one antibody fragment (14) is an Fab fragment, in particular an Fab fragment of the molecules IgG, IgA, IgM and/or IgE.

7. Method according to any of Claims 1 to 6, **characterized in that** a first detection antibody (23) which specifically binds to the ligands (2) of the first type of ligand and is labelled with a first marker is contacted with the first test site (8), that markers not bound to an immobilized receptor (9, 11) are subsequently removed from the substrate (7), and that the first measured signal is then generated as a function of the concentration of the first marker.

8. Method according to any of Claims 1 to 7, **characterized in that** a second detection antibody (24) which specifically binds to the ligands (3) of the second type of ligand and is labelled with a second marker is contacted with the second test site (10), that markers not bound to an immobilized receptor (9, 11) are subsequently removed from the substrate (7), and that the second measured signal is then generated as a function of the concentration of the second marker and the concentration of the at least one antibody fragment (14) in the mixture.

9. Method according to any of Claims 1 to 8, **characterized in that** the first marker and/or the second marker are enzymes/is an enzyme, that recording of the measured signals involves contacting the enzyme with at least two chemicals, between which a chemical redox reaction occurs in the presence of said enzyme, and that the first measured signal and/or the second measured signal are/is generated by measuring a redox potential.

10. Method according to any of Claims 1 to 9, **characterized in that** recording of the measured signals involves illuminating the first marker and/or the second marker with an exciting radiation which stimulates the marker(s) to emit a luminescent radiation, and that the first measured signal and/or the second measured signal are/is generated by measuring the luminescent radiation of the relevant marker.

11. Method according to any of Claims 1 to 10, **characterized in that** recording of the measured signals involves contacting the first test site (8) and/or the second test site (10) with a chemiluminescence substrate in which a luminescent radiation is stimulated as a function of the ligands (2) of the first type of ligand binding to the first receptor (9) and/or as a function of the ligands (3) of the second type of ligand binding to the second receptor (11), and that the first measured signal and/or the second measured signal are/is generated by measuring the luminescent radiation at the relevant test site (8, 10).

12. Device (1) for detemining the concentrations of ligands (2, 3) of different types of ligand in a sample to be analysed, comprising a measurement chamber (4) having at least one inlet port (5) and one outlet port (6), which chamber has a substrate (7) on which a first receptor (9) of a first type of receptor has been immobilized at a first test site (8) and a second receptor (11) of a second type of receptor has been immobilized at a second test site (10), wherein the first type of receptor specifically binds to at least one first binding site (18) of the ligands (2) of the first type of ligand and the second type of receptor (11) specifically binds to at least one second binding site (19) of the ligands (3) of the second type of ligand, wherein a first sensor (15) for recording a first measured signal which is a function of the concentration of the ligand (2) bound to the first receptor (9) is assigned to the first test site (8) and a second sensor (16) for recording a second measured signal which is a function of the concentration of the ligand (3) bound to the second receptor (11) is assigned to the second test site (10), **characterized in that** at least one non-immobilized monovalent antibody fragment (14) which specifically binds to the second binding site (19) and upon contact with the sample to be analysed forms therewith a mixture containing a predetermined concentration of said antibody fragment (14) is arranged in the measurement chamber (4), and that the second sensor (16) is connected to an evaluation apparatus which is designed for determining the concentration of the second ligand (3) in the sample from the second measured signal and the predetermined concentration of the antibody fragment (14) in the mixture.

13. Device according to Claim 12, **characterized in that** the antibody fragment (14) is stabilized by way of a gel-like, pasty or solid form in the measurement chamber (4), preferably so as to adhere to a boundary wall of the measurement chamber (4).

14. Device (1) according to the preamble of Claim 12, **characterized in that** the device has a mixing apparatus (26) which, in order to mix the sample with at least one monovalent antibody fragment (14) which specifically binds to the second binding site (19), is connected to a feed opening (22) for the sample and a receiving space (27) containing the antibody fragment (14), that the mixing apparatus (26) has a discharge opening for the mixture produced from the sample and the antibody fragment (14), which opening is connected to the inlet port (5) of the measurement chamber (4), that the mixing apparatus (26) is developed so as for the mixture to contain a predetermined concentration of the antibody fragment (14), and that the second sensor (16) is connected to an evaluation apparatus which is designed for determining the concentration of the second ligand (3) in the sample from the second measured signal and the concentration of the antibody fragment (14) in the mixture.

15. Device (1) according to any of Claims 12 to 14, **characterized in that** the antibody fragment (14) corresponds to a fragment of the second receptor.

16. Device (1) according to any of Claims 12 to 15, **characterized in that** the at least one antibody fragment (14) is a fragment of a polyclonal antibody.

17. Device (1) according to any of Claims 12 to 16, **characterized in that** the at least one antibody fragment (14) is a fragment of a monoclonal antibody.

18. Device (1) according to any of Claims 12 to 17, **characterized in that** the at least one antibody fragment (14) is an Fab fragment, in particular an Fab fragment of the molecules IgA, IgM and/or IgE.

19. Device (1) according to any of Claims 14 to 18, **characterized in that** the antibody fragment (14) is stabilized by way of a gel-like, pasty or solid form in the receiving space (27), preferably so as to adhere to a boundary wall of the receiving space (27), and that the receiving space (27) is designed by way of a flow mixing chamber for dissolving the antibody fragment (14) in the sample, which flow mixing chamber connects the feed opening (22) for the sample to the inlet port (5) of the measurement chamber (4).

20. Device (1) according to any of Claims 14 to 19, **characterized in that** the flow mixing chamber has a mixer structure (12) which is developed such that the mixture, upon passing through the flow mixing chamber, is deflected preferably in alternately opposite directions, and that the mixer structure (12) is arranged between the antibody fragment (14) which is in a gel-like, pasty or solid form and the inlet port (5) of the measurement chamber.

21. Device (1) according to any of Claims 12 to 20, **characterized in that** the sensors (15, 16) are optical sensors, and that the first receptor (9) is arranged preferably directly on the first sensor (15) for detecting a first luminescent radiation generated as a function of the ligands (2) of the first type of ligand binding to the first receptor (9) and/or the second receptor (11) is arranged preferably directly on the second sensor (16) for detecting a second, luminescent radiation generated as a function of the ligands (3) of the second type of ligand binding to the second receptor (11).

22. Kit for carrying out the method according to any of Claims 1 to 11, comprising
- a device (1) according to any of Claims 12 to 21, in which the first sensor (15) is designed by way of a sensor for measuring a redox potential,
- a detection antibody (23) labelled with an enzyme, which antibody specifically binds to ligands (2) of the first type of ligand, and
- at least two chemicals, between which a chemical redox reaction occurs upon contact with said enzyme.

23. Kit for carrying out the method according to any of Claims 1 to 11, comprising
- a device (1) according to any of Claims 12 to 21, in which the second sensor (16) is designed by way of a sensor for measuring a redox potential,
- a detection antibody (24) labelled with an enzyme, which antibody specifically binds to ligands (3) of the second type of ligand, and
- at least two chemicals, between which a chemical redox reaction occurs upon contact with said enzyme.

24. Kit for carrying out the method according to any of Claims 1 to 11, in particular according to Claim 23, comprising
- a device (1) according to any of Claims 12 to 21,
- a detection antibody labelled with an enzyme, which antibody specifically binds to ligands of the first type of ligand (2), and
- a chemiluminescence substrate in which contact with said enzyme triggers a chemical reaction, during which a luminescent radiation is released which the first sensor (15) is sensitive to.

25. Kit for carrying out the method according to any of Claims 1 to 11, in particular according to Claim 23, comprising
- a device (1) according to any of Claims 12 to 21,
- a detection antibody (24) labelled with an enzyme, which antibody specifically binds to ligands (3) of the second type of ligand, and
- a chemiluminescence substrate in which contact with said enzyme triggers a chemical reaction, during which a luminescent radiation is released which the second sensor (16) is sensitive to.

26. Kit for carrying out the method according to any of Claims 1 to 11, in particular according to Claim 23 or 25, comprising
- a detection antibody (23) which specifically binds to ligands of the first type of ligand and which is labelled with a marker which can be stimulated by illumination with an exciting radiation to emit a luminescent radiation,
- a device (1) according to any of Claims 12 to 20, in which the first sensor (15) is sensitive to said luminescent radiation and insensitive to said exciting radiation, and
- a radiation source which is arranged for sending out the exciting radiation to the first test site (8).

27. Kit for carrying out the method according to any of Claims 1 to 11, in particular according to Claim 22 or 24, comprising
- a detection antibody (23) which specifically binds to ligands (2) of the first type of ligand and which is labelled with a marker which can be stimulated by illumination with an exciting radiation to emit a luminescent radiation,
- a device (1) according to any of Claims 12 to 20, in which the second sensor (16) is sensitive to said luminescent radiation and insensitive to said exciting radiation, and
- a radiation source which is arranged for sending out the exciting radiation to the second test site (10).

## Revendications

1. Procédé pour la détermination des concentrations de ligands (2, 3) dans un échantillon à analyser, l'échantillon présentant au moins un ligand (2) d'un premier type de ligands, qui présente au moins un premier site de liaison (18) pour lequel les récepteurs (9) d'un premier type de récepteurs sont spécifiques en liaison, l'échantillon présentant au moins un ligand (3) d'un deuxième type de ligands qui présente au moins un deuxième site de liaison (19), pour lequel les récepteurs (11) d'un deuxième type de récepteurs sont spécifiques en liaison, où au moins un premier récepteur (9) du premier type de récepteurs est immobilisé en au moins un premier site de test (8) et au moins un deuxième récepteur (11) du deuxième type de récepteurs est immobilisé en au moins un deuxième site de test (10) sur un substrat (7), au moins un fragment d'anticorps (14) étant mis à disposition, qui est spécifique en liaison pour le deuxième site de liaison (19), où ledit au moins un fragment d'anticorps (14) est mélangé avec l'échantillon de manière telle qu'il se trouve en une concentration prédéfinie dans le mélange ainsi obtenu, le mélange étant ensuite mis en contact, en vue de la liaison des ligands (2, 3) sur les récepteurs (9, 11), avec le substrat (7) de manière telle qu'au moins un ligand (2) du premier type de ligands peut se lier audit au moins un premier récepteur (9) et au moins un ligand (3) du deuxième type de ligands peut se lier audit au moins un deuxième récepteur (11), les constituants du mélange qui ne sont pas liés à un récepteur immobilisé (9, 11) étant éliminés du substrat (7), un premier signal de mesure, dépendant de la concentration du ligand (2) lié au premier récepteur (9) et un deuxième signal de mesure dépendant de la concentration du ligand (3) lié au deuxième récepteur (11) étant obtenus, où on détermine, à l'aide du premier signal de mesure la concentration du ligand (2) du premier type de ligands et la concentration du ligand (3) du deuxième type de ligands à l'aide du deuxième signal de mesure et la concentration dudit au moins un fragment d'anticorps (14) dans le mélange, **caractérisé en ce que** ledit au moins un fragment d'anticorps (14) est monovalent.

2. Procédé selon la revendication 1, **caractérisé en ce qu'**au moins le ligand (3) du deuxième type de ligands présente au moins deux sites de liaison (19) pour les fragments d'anticorps (14).

3. Procédé selon la revendication 1 ou 2, **caractérisé en ce que** le fragment d'anticorps (14) correspond à un fragment du deuxième récepteur (11).

4. Procédé selon l'une quelconque des revendications 1 à 3, **caractérisé en ce que** ledit au moins un fragment d'anticorps (14) est un fragment d'un anticorps polyclonal.

5. Procédé selon l'une quelconque des revendications 1 à 4, **caractérisé en ce que** ledit au moins un fragment d'anticorps (14) est un fragment d'un anticorps monoclonal.

6. Procédé selon l'une quelconque des revendications 1 à 5, **caractérisé en ce que** ledit au moins un fragment d'anticorps (14) est un fragment Fab, en particulier un fragment Fab des molécules IgG, IgA, IgM et/ou IgE.

7. Procédé selon l'une quelconque des revendications 1 à 6, **caractérisé en ce qu'**on met en contact un premier anticorps de détection (23) spécifique en liaison pour les ligands (2) du premier type de ligands, marqué par un premier marqueur, avec le premier site de test (8), **en ce qu'**ensuite, les marqueurs non liés à un récepteur immobilisé (9, 11) sont éliminés du substrat (7) et **en ce qu'**on produit ensuite le premier signal de mesure en fonction de la concentration du premier marqueur.

8. Procédé selon l'une quelconque des revendications 1 à 7, **caractérisé en ce qu'**on met en contact un deuxième anticorps de détection (24) spécifique en liaison pour les ligands (3) du deuxième type dé ligands, marqué par un deuxième marqueur, avec le deuxième site de test (10), **en ce qu'**ensuite, les marqueurs non liés à un récepteur immobilisé (9, 11) sont éliminés du substrat (7) et **en ce qu'**on produit ensuite le deuxième signal de mesure en fonction de la concentration du deuxième marqueur et de la concentration dudit au moins un fragment d'anticorps (14) dans le mélange.

9. Procédé selon l'une quelconque des revendications 1 à 8, **caractérisé en ce que** le premier marqueur et/ou le deuxième marqueur est/sont une enzyme, **en ce que** l'enzyme est mise en contact, pendant l'enregistrement des signaux de mesure, avec au moins deux produits chimiques, entre lesquels il se déroule une réaction chimique d'oxydoréduction en présence de l'enzyme et **en ce que** le premier signal de mesure et/ou le deuxième signal de mesure est/sont produit(s) par la mesure d'un potentiel d'oxydoréduction.

10. Procédé selon l'une quelconque des revendications 1 à 9, **caractérisé en ce que** le premier marqueur et/ou le deuxième marqueur est/sont irradié(s) pendant l'enregistrement des signaux de mesure par un rayonnement d'excitation, qui excite le/les marqueur(s) pour émettre un rayonnement luminescent et **en ce que** le premier signal de mesure et/ou le deuxième signal de mesure est/sont produit(s) par mesure du rayonnement de luminescence du marqueur concerné.

11. Procédé selon l'une quelconque des revendications 1 à 10, **caractérisé en ce que** le premier site de test (8) et/ou le deuxième site de test (10) est/sont mis en contact pendant l'enregistrement des signaux de mesure avec un substrat de chimiluminescence, dans lequel un rayonnement de luminescence est excité en fonction de la liaison des ligands (2) du premier type de ligands sur le premier récepteur (9) et/ou en fonction de la liaison des ligands (3) du deuxième type de ligands sur le deuxième récepteur (11) et **en ce que** le premier signal de mesure et/ou le deuxième signal de mesure est/sont produit (s) par la mesure du rayonnement de luminescence sur le site de test (8, 10) concerné.

12. Dispositif (1) pour la détermination des concentrations de ligands (2, 3) de types de ligands différents dans un échantillon à analyser, présentant une chambre de mesure (4) avec au moins une ouverture d'admission (5) et une ouverture d'évacuation (6) qui contient un substrat (7) sur lequel sont immobilisés, sur un premier site de test (8), un premier récepteur (9) d'un premier type de récepteurs et, sur un deuxième site de test (10) un deuxième récepteur (11) d'un deuxième type de récepteurs, le premier type de récepteurs étant-spécifique en liaison pour au moins un premier site de liaison (18) des ligands (2) du premier type de ligands et le deuxième type de récepteurs (11) étant spécifique en liaison pour au moins un deuxième site de liaison (19) des ligands (3) du deuxième type de ligands, un premier capteur (15) étant associé au premier site de test (8) pour l'enregistrement d'un premier signal de mesure dépendant de la concentration du ligand (2) lié au premier récepteur (9) et un deuxième capteur (16) étant associé au deuxième site de test (10) pour enregistrer un deuxième signal de mesure dépendant de la concentration du ligand (3) lié au deuxième récepteur (11), **caractérisé en ce qu'**on a disposé dans la chambre de mesure (4) au moins un fragment d'anticorps (14) monovalent non immobilisé, qui est spécifique en liaison pour le deuxième site de liaison (19) et qui forme lors d'un contact avec l'échantillon à analyser un mélange avec celui-ci, qui contient le fragment d'anticorps (14) en une concentration prédéfinie et **en ce que** le deuxième capteur (16) est relié à un dispositif d'évaluation, qui est réalisé pour la détermination de la concentration du deuxième ligand (3) dans l'échantillon à partir du deuxième signal de mesure et de la concentration prédéfinie du fragment d'anticorps (14) dans le mélange.

13. Dispositif selon la revendication 12, **caractérisé en ce que** le fragment d'anticorps (14) est stabilisé sous forme de gel, pâteuse ou solide dans la chambre (4) de préférence de manière telle qu'il adhère à une paroi de délimitation de la chambre de mesure (4).

14. Dispositif (1) selon le préambule de la revendication 12, **caractérisé en ce que** le dispositif présente un dispositif de mélange (26) qui est relié, pour le mélange de l'échantillon avec au moins un fragment d'anticorps (14) monovalent spécifique en liaison pour le deuxième site de liaison (19) avec une ouverture d'alimentation (22) pour l'échantillon et une chambre de réception (27) contenant le fragment d'anticorps (14), **en ce que** le dispositif de mélange (26) présente une ouverture d'évacuation pour le mélange formé à partir de l'échantillon et du fragment d'anticorps (14), qui est reliée avec l'ouverture d'admission (5) de la chambre de mesure (4), **en ce que** le dispositif de mélange (26) est réalisé de manière telle que le fragment d'anticorps (14) se trouve en une concentration prédéfinie dans le mélange et **en ce que** le deuxième capteur (16) est relié à un dispositif d'évaluation qui est réalisé pour la détermination de la concentration du deuxième ligand (3) dans l'échantillon à partir du deuxième signal de mesure et de la concentration du fragment d'anticorps (14) dans le mélange.

15. Dispositif (1) selon l'une quelconque des revendications 12 à 14, **caractérisé en ce que** le fragment d'anticorps (14) correspond à un fragment du deuxième récepteur.

16. Dispositif (1) selon l'une quelconque des revendications 12 à 15, **caractérisé en ce que** ledit au moins un fragment d'anticorps (14) est un fragment d'un anticorps polyclonal.

17. Dispositif (1) selon l'une quelconque des revendications 12 à 16, **caractérisé en ce que** ledit au moins un fragment d'anticorps (14) est un fragment d'un anticorps monoclonal.

18. Dispositif (1) selon l'une quelconque des revendications 12 à 17, **caractérisé en ce qu'**au moins un fragment d'anticorps (14) est un fragment Fab, en particulier un fragment Fab des molécules IgA, IgM et/ou IgE.

19. Dispositif (1) selon l'une quelconque des revendications 14 à 18, **caractérisé en ce que** le fragment d'anticorps (14) est stabilisé sous forme de gel, pâteuse ou solide dans la chambre de réception (27) de préférence de manière telle qu'il adhère à une paroi de délimitation de la chambre de réception (27) et **en ce que** la chambre de réception (27) est réalisée, pour dissoudre le fragment d'anticorps (14) dans l'échantillon, sous forme de chambre de mélange à flux, via laquelle l'ouverture d'alimentation (22) pour l'échantillon est reliée à l'ouverture d'admission (5) de la chambre de mesure (4).

20. Dispositif (1) selon l'une quelconque des revendications 14 à 19, **caractérisé en ce que** la chambre de mélange à flux présente une structure de mélangeur (12) qui est réalisée de manière telle que le mélange, lorsqu'il s'écoule dans la chambre de mélange à flux, est dévié de préférence alternativement dans des directions opposées l'une à l'autre et **en ce que** la structure de mélange (12) est disposée entre le fragment d'anticorps (14) sous forme de gel, de pâte ou solide et l'ouverture d'admission (5) de la chambre de mesure.

21. Dispositif (1) selon l'une quelconque des revendications 12 à 20, **caractérisé en ce que** les capteurs (15, 16) sont des capteurs optiques et **en ce que** le premier récepteur (9) est disposé de préférence directement sur le premier capteur (15) pour la détection d'un rayonnement de luminescence produit en fonction de la liaison du ligand (2) du premier type de ligands sur le premier récepteur (9) et/ou le deuxième récepteur (11) est disposé de préférence directement sur le deuxième capteur (16) pour la détection d'un deuxième rayonnement de luminescence produit en fonction de la liaison des ligands (3) du deuxième type de ligands sur le deuxième récepteur (11).

22. Kit pour la réalisation du procédé selon l'une quelconque des revendications 1 à 11, comprenant
- un dispositif (1) selon l'une quelconque des revendications 12 à 21, dans lequel le premier capteur (15) est un capteur conçu pour la mesure d'un potentiel d'oxydoréduction,
- un anticorps de détection (23) spécifique en liaison pour les.ligands (2) du premier type de ligands, marqué par une enzyme et
- au moins deux produits chimiques, entre lesquels il se produit une réaction chimique d'oxydoréduction lors du contact avec l'enzyme.

23. Kit pour la réalisation du procédé selon l'une quelconque des revendications 1 à 11, comprenant
- un dispositif (1) selon l'une quelconque des revendications 12 à 21, dans lequel le deuxième capteur (16) est un capteur conçu pour la mesure d'un potentiel d'oxydoréduction,
- un anticorps de détection (24) spécifique en liaison pour les ligands (3) du deuxième type de ligands, marqué par une enzyme et
- au moins deux produits chimiques, entre lesquels il se produit une réaction chimique d'oxydoréduction lors du contact avec l'enzyme.

24. Kit pour la réalisation du procédé selon l'une quelconque des revendications 1 à 11, en particulier selon la revendication 23, comprenant
- un dispositif (1) selon l'une quelconque des revendications 12 à 21,
- un anticorps de détection spécifique en liaison pour les ligands du premier type de ligands (2), marqué par une enzyme et
- un substrat de chimiluminescence, dans lequel une réaction chimique est déclenchée lors d'un contact avec l'enzyme, lors de laquelle un rayonnement de luminescence est libéré auquel le premier capteur (15) est sensible.

25. Kit pour la réalisation du procédé selon l'une quelconque des revendications 1 à 11, en particulier selon la revendication 23, comprenant
- un dispositif (1) selon l'une quelconque des revendications 12 à 21,
- un anticorps de détection (24) spécifique en liaison pour les ligands (3) du deuxième type de ligands, marqué par une enzyme et
- un substrat de chimiluminescence, dans lequel une réaction chimique est déclenchée lors d'un contact avec l'enzyme, lors de laquelle un rayonnement de luminescence est libéré auquel le deuxième capteur (16) est sensible.

26. Kit pour la réalisation du procédé selon l'une quelconque des revendications 1 à 11, en particulier selon la revendication 23 ou 25, comprenant
- un anticorps de détection (23) spécifique en liaison pour les ligands du premier type de ligands qui est marqué par un marqueur qui est excitable par irradiation par un rayonnement d'excitation pour l'émission d'un rayonnement de luminescence,
- un dispositif (1) selon l'une quelconque des revendications 12 à 20, dans lequel le premier capteur (15) est sensible au rayonnement de luminescence et insensible au rayonnement d'excitation, et
- une source de rayonnement, qui est disposée pour émettre le rayonnement d'excitation sur le premier site de test (8).

27. Kit pour la réalisation du procédé selon l'une quelconque des revendications 1 à 11, en particulier selon la revendication 22 ou 24, comprenant
- un anticorps de détection (23) spécifique en liaison pour les ligands (2) du premier type de ligands qui est marqué par un marqueur qui est excitable par irradiation par un rayonnement d'excitation pour l'émission d'un rayonnement de luminescence,
- un dispositif (1) selon l'une quelconque des revendications 12 à 20, dans lequel le deuxième capteur (16) est sensible au rayonnement de luminescence et insensible au rayonnement d'excitation, et
- une source de rayonnement, qui est disposée pour émettre le rayonnement d'excitation sur le deuxième site de test (10).
